# EUROPEAN PATENT SPECIFICATION

(11) **EP 1 945 807 B1**
(45) Date of publication and mention of the grant of the patent: **13.01.2010**
(21) Application number: 06808729.5
(22) Date of filing: 01.11.2006
(51) Int. Cl.: C12Q 1/68, G01N 27/447, G06F 19/00

(54) **IMPROVEMENTS IN SEQUENCING**
VERBESSERUNGEN BEI DER SEQUENZIERUNG
AMELIORATIONS APPORTEES AU SEQUENCAGE

(30) Priority: 02.11.2005 GB 0522335
(43) Date of publication of application: 23.07.2008
(73) Proprietor: Deltadot Limited, London SW7 2BW (GB)
(72) Inventor: ISAACS, David, London SW7 2BW (GB)
(74) Representative: Williams, Gareth Owen
(86) International application number: PCT/GB2006/050365
(87) International publication number: WO 2007/052078

(56) References cited:
- WO-A-98/00708
- US-A- 5 098 536
- US-A1- 2002 138 205
- US-A1- 2003 203 408
- US-B1- 6 397 150

## Description

### FIELD OF THE INVENTION

The present invention relates to improvements in sequencing of polymers. In preferred embodiments, the invention relates to the use of termination artefacts arising from a chain termination sequencing method as internal markers. In certain aspects, the invention relates to the sequencing of nucleic acids.

### BACKGROUND TO THE INVENTION

Chain termination sequencing of DNA is a commonly used method for determining the order of bases within a nucleic acid polymer. As described by Sanger et al (Proc Natl Acad Sci USA 1977, 74(12): 5463-7) the method relies on the incorporation of modified bases into a DNA polymerase reaction. The modified bases, typically dideoxynucleotide triphosphates (ddNTPs), are included in a polymerase reaction mix together with unmodified dNTPs, a template DNA strand, and a primer strand. The primer hybridises to a complementary portion of the template strand, and the DNA polymerase interacts with the primer and template strand to extend the primer by addition of complementary dNTPs. When a ddNTP is incorporated into the growing strand, the polymerase is no longer able to add further dNTPs to the strand, and the chain extension terminates.

By performing the chain termination with a single type of ddNTP (say ddTTP), together with the corresponding dNTP (for example dTTP) and the other three dNTPs, a mixture of chain lengths will be obtained, all of which terminate with a ddTTP at an appropriate position. When this mixture is fractionated by electrophoresis the pattern of bands will show the distribution of dT in the synthesised fragments. Repeating this process for each of the other three ddNTPs allows the complete sequence of bases to be read.

Generally the synthesised strands will be labelled in some way, to permit ready detection of the fragments. Originally radionucleotides were included in the polymerase reaction, but more recently fluorescent labels have been used. Conventionally a single type of label is used, and all four reactions are fractionated separately, for example as four lanes on an electrophoretic gel.

More recently, four different labels have been used, allowing all reactions to be fractionated simultaneously in a single lane. This permits automation of the process, and allows a rapid throughput. However, the need to use four different labels adds to the cost and complexity of the process.

International patent application WO96/35946, describes a method for detecting polymers, including nucleic acids, based on measuring changes in absorbance of light of a certain wavelength as the polymer passes a light emitter and detector. Where the polymer is DNA, ultraviolet light is typically used, as DNA absorbs light having a wavelength in the 220 to 290 nm range. This process is known as label free intrinsic imaging, as no extrinsic label needs to be incorporated into the polymer. The referenced application also suggests that label free intrinsic imaging may be used in DNA sequencing. Given that no label is used, it is apparent that the sequencing reactions must be fractionated in four separate lanes.

Where a single label is used in all four reactions, in order to take account of possible differences in migration pattern between lanes, markers of known size are typically incorporated into the fractionation steps. These markers are then aligned between lanes, and used to calibrate the fractionations, to ensure that the sequences are read in the correct order. Addition of markers, and calibration, adds to the cost and complexity of the sequencing process.

International patent application WO02/12877, describes an analysis system and method which can be used in the sequencing of polymers such as DNA. The system is based on the label free intrinsic imaging system described above, and allows the classification of groups of migrating polymers into common sets. As polymer bands are made to migrate by electrophoresis past a UV detector, their velocity is calculated. An equiphase space-time map is generated from the velocities, and a vertex finder used to identify at least one vertex from the map. A single vertex is found for each group (for example, those bands having a common starting position). The grouping of bands can then be used to separate a plurality of initial groups from a single electrophoresis run. As described in the referenced application, this can allow four chain termination sequencing reactions to be run in a single electrophoretic lane, simply by separating the introduction of the four reactions into the lane either in time (for example, by adding the reaction mixes in sequence to the lane) or in space (by introducing the reaction mixes at distinct locations along the lane). While this method permits a single lane to be used even when no labels are incorporated into the reaction, the use of markers to calibrate the electrophoretic fractionations is still required.

US-B1-6 397 150 discloses a method of sequencing a polymer comprising the data-analysis steps of: (i) providing a plurality of data-sets from a plurality of chain termination reactions; (ii) aligning two or more of the data-sets using traces from a calibrant added to the reactions; (iii) determining the polymer sequence using the aligned data.

The present inventors have determined a method whereby intrinsic information from the electrophoresis may be used as an intrinsic marker, so removing the need to introduce a separate marker.

In Sanger (chain germination) sequencing DNA fragments terminated by a specific ddNTP will be seen at the highest concentration. For any given track there will also be lower concentration fragments in the mix that are generally considered undesirable. For example, those where the DNA polymerase has fallen off the template DNA before it has included a terminating ddNTP residue. These so called false stops will generally be noticed only when all DNA in the mix is being labelled, or in the case of label free systems, visualised through UV absorbance. When visualised as intensity peaks, the false stops appear smaller than the "real peaks" terminated by a ddNTP, due to the lower concentration.

The present inventors have found that there are consistent correlations of artefact peak heights and shapes (concentrations) across fractionations. These occur at the same peak position through different runs/experiments for the same sequence template, implying that the formation of false stops is a non random process. This consistency can be used to enhance sequencing capabilities as the false stops can be used as an intrinsic marker system allowing the four tracks representing the four nucleotide fragment termination series to be aligned by identifying corresponding artefact peaks. It is of note that previous work using fluorescently labelled primers failed to either see the significance of these peaks, or were not able to see that these peaks are consistent in relative intensity across different experiments as the information was obscured by the label.

### SUMMARY OF THE INVENTION

The present invention provides methods and systems of use in the sequencing of polymers using a chain termination method, using germination artefacts as intrinsic markers for the sequencing.

According to a first aspect of the present invention, there is provided a method of sequencing a polymer, the method comprising
providing a plurality of data sets, each set comprising data representing the concentration of synthesised polymers from a plurality of chain termination reactions, wherein the data sets include termination artefacts;
aligning two or more of the data sets based on at least one termination artefact present in said two or more data sets; and
determining the polymer sequence based on the aligned data.

Thus, the termination artefacts may be used as intrinsic markers to permit alignment of two or more data sets derived from chain germination reactions. In this way the reliability and ease of sequencing can be improved without the need to introduce extrinsic markers.

Preferably the polymers are nucleic acids, more preferably DNA. The chain termination reactions are preferably dideoxynucleotide triphosphate (ddNTP) termination reactions, although any suitable chain termination reaction may be used. Where nucleic acids are being sequenced, preferably data from at least four chain termination reactions is provided, each reaction being performed with a different type of ddNTP or other terminator.

The polymers may be labelled, but are preferably unlabelled. The concentration of the polymers may be represented by the detected intensity of light or other radiation detected from the polymers; preferably the concentration is represented by the detected absorption of UV light by the polymers.

Preferably the termination artefacts are false stops; that is, polymer fragments the synthesis of which has terminated before the incorporation of a chain terminating monomer.

Preferably each data set represents the concentration of synthesised polymers separated according to a physical characteristic. The data set will then include data representing the location of the different polymers within a data set; this may be an absolute location (for example, absolute displacement along an electrophoresis track), or a relative location (order of different polymers within the set, time that each polymer was detected, or separation between polymers along an electrophoresis track). The polymers may be fractionated according to their chain length. The chain length may be indirectly used to fractionate the polymers; for example, where the electric charge of the polymer is proportional to its chain length, and the polymers are fractionated by charge. Two or more of the data sets may be obtained from the same separation (for example, four chain termination reactions separated in a single fractionation lane), or all the data sets may be obtained from separate separations (such as four chain termination reactions separated in four fractionation lanes). Where the data sets are obtained from the same separation, they may conveniently be derived from a single data set by the method described in WO02/12877, or a similar method.

Preferably each of said plurality of data sets is aligned with one another.

The step of aligning the data sets may comprise determining the location of at least one termination artefact present in at least two data sets, and transforming the data sets such that the termination artefacts are present in the same location in each transformed data set. Preferably a plurality of termination artefacts are used. Preferably the termination artefacts are present in more than two data sets, although each termination artefact will not necessarily be present in all the data sets. Cumulative alignment techniques may also be used; that is, a first termination artefact may be used to align first and second data sets, and a second artefact used to align second and third data sets, such that all three data sets are aligned. This process may be repeated to align greater numbers of data sets. In addition, or instead, additional termination artefacts may be used to refine the alignment of data sets which have already been aligned with initial artefacts.

Preferably the method comprises the step of generating the plurality of data sets. This may take the form of detecting the concentration of synthesised polymers from a chain termination reaction, and including a data item in the data set representing that concentration. The concentration may be detected by causing the polymers to pass between a light source and a light detector; preferably a UV source and detector. Preferably the polymers are size fractionated before or while they are caused to pass between the source and detector. This may be effected by means of electrophoresis, or any other suitable technique. Fractionation of the polymers may comprise passing the polymers through a matrix; the matrix may be solid or liquid. A preferred matrix is polyethylene oxide (PEO), although other matrices may be used. The polymers may pass through the matrix while under an electric field.

The data sets may of course be generated in any suitable manner.

The method preferably comprises the steps of fractionating synthesised polymers; passing the fractionated polymers over a detector arranged to detect the concentration of said polymers; and generating a data set representing the concentration of the fractionated polymers.

Preferably the method comprises the step of performing one or more chain termination reactions in order to obtain a plurality of synthesised polymers. Suitable chain termination reactions are known to those of skill in the art.

A further aspect of the invention provides a method of sequencing a nucleic acid, the method comprising
providing a plurality of data sets, each set comprising data representing the concentration of synthesised nucleic acids from a plurality of chain termination reactions, each reaction being performed with a different termination nucleotide, wherein the data sets include termination artefacts;
aligning two or more of the data sets based on at least one termination artefact present in said two or more data sets; and
determining the nucleic acid sequence based on the aligned data.

Preferably the nucleic acids are DNA. Preferably four data sets are provided, each representing a chain termination reaction performed with a different one of the four nucleotides found in nuclei acids, or corresponding modified nucleotides.

Also provided is an apparatus for sequencing polymers, the apparatus comprising
detection means for detecting the concentration of polymers within a plurality of sets of synthesised polymers from a plurality of chain termination reactions;
a data processing means programmed to execute computer software to derive a plurality of data sets containing data corresponding to said plurality of sets of synthesised polymers;
align two or more of the data sets based on at least one termination artefact present in said two or more data sets;
and
output a polymer sequence based on the adjusted data.

The detection means may comprise a light emitter and sensor arranged such that said polymers interrupt the path between the emitter and sensor. The light is preferably UV light.

Preferably the apparatus further comprises a separation channel along which said polymers may be moved and separated. The channel is preferably disposed adjacent the detection means. Conveniently the channel is arranged to move separated polymers past the detection means. The channel may be an electrophoresis channel, and may be in the form of a capillary or microfluidic chip.

The apparatus may comprise a plurality of separation channels, although preferably only one channel is provided. Where a single channel is provided, there may be a plurality of openings whereby said plurality of sets of synthesised polymers may be separately introduced into the channel. Alternatively, a single opening may be provided, and said plurality of sets may be introduced sequentially.

According to a further aspect of the present invention, there is provided a method of correlating a plurality of chain termination reactions, the method comprising
providing a plurality of data sets, each set comprising data representing the concentration of synthesised polymers from a plurality of chain termination reactions, wherein the data sets include termination artefacts; and
aligning two or more of the data sets based on at least one termination artefact present in said two or more data sets, to correlate the plurality of reactions.

The comparison of termination artefacts may be achieved by aligning two or more of the data sets based on at least one termination artefact present in said two or more data sets; and comparing the aligned data sets.

The present invention further provides the use of termination artefacts in a method of sequencing polymers; in a method of aligning data sets representing chain termination reactions; or in a method of correcting data from chain termination reactions. Also provided is the use of termination artefacts in a method of quality control of a polymerase enzyme.

### BRIEF DESCRIPTION OF THE FIGURES

These and other aspects of the present invention will now be described by way of example only and with reference to the accompanying drawings, in which:
Figure 1 shows an illustration of the Sanger chain termination method for sequencing DNA;
Figure 2 shows a DNA sequencing platform making use of label free intrinsic imaging, and of the algorithms described in WO02/12877;
Figure 3 illustrates the generation of 'false stop' termination artefacts in the Sanger chain termination method;
Figure 4 shows artefact peaks obtained from the sequencing platform of Figure 2;
Figure 5 illustrates the use of such artefact peaks as intrinsic markers for alignment of sequencing reactions; and
Figure 6 shows extended track alignments generated from a plasmid sequencing experiment.

### DETAILED DESCRIPTION OF THE INVENTION

The present invention makes use of the realisation that termination artefacts created during the chain termination sequencing method are consistent between reactions performed on the same template with the same polymerase. We have determined that these artefacts may thus be used as intrinsic markers for alignment of sequencing reactions. Here we describe the background to the Sanger chain termination sequencing method, along with the label free intrinsic imaging system used by the present inventors. We then illustrate how the artefacts may be used to align sequencing runs.

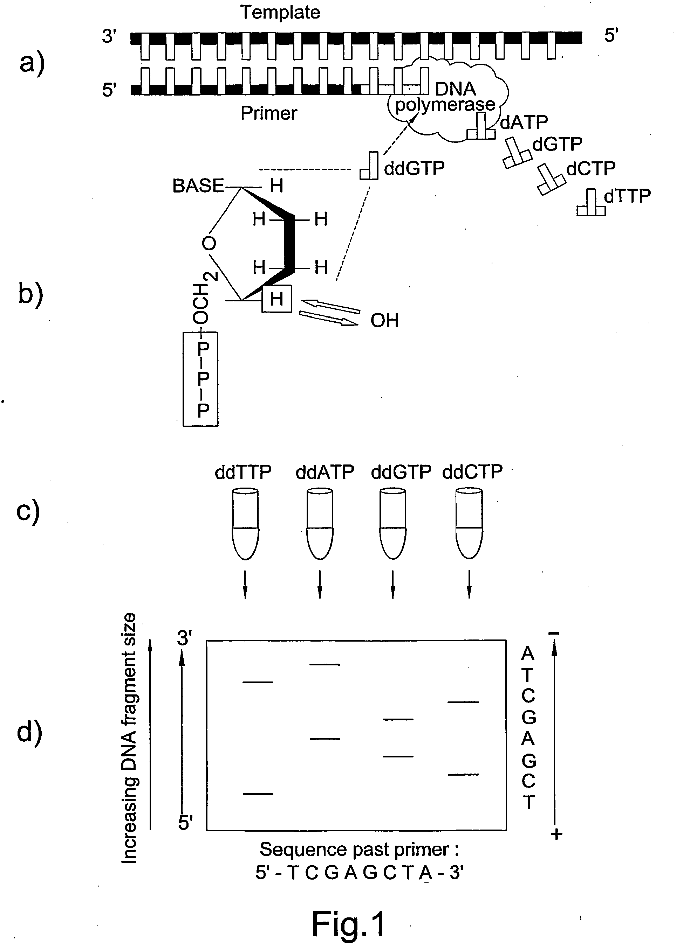

Referring first of all to Figure 1, this illustrates the Sanger chain termination method. Although there are many variations of the method, the basic principle remains the same.

Figure 1a. An oligonucleotide known as a primer is specifically designed to anneal to a complementary section of DNA template. The DNA template is single stranded, having been chemically or thermally denatured. An enzyme, DNA polymerase, extends the complimentary strand in a 5' to 3' direction. Nucleotides consisting of the four DNA bases adenine (dATP), thymine (dTTP), guanine (dGTP) and cytosine (dCTP), are added in the reaction mix in order to extend the growing DNA chain. Additionally, an analogue of a dNTP, called a dideoxynucleotide (ddNTP), represented as ddGTP in our example, is added to the reaction mix. The ddNTP prevents the DNA polymerase from extending the growing DNA chain in the 3' direction. This results in a population of truncated DNA fragments of varying lengths terminated by the ddNTP.

Figure 1b. The chemical structure of a ddNTP can be seen in this figure, the ribose moiety lacks the hydroxyl group found in a dNTP. This is necessary for forming a phosphodiester bond with the next incoming dNTP (phosphate is represented by a "P").

Figure 1c. Four chain termination reactions are carried out separately, each reaction utilising the random incorporation of one of the four ddNTPs (ddTTP, ddATP, ddGTP, ddCTP). The DNA fragments generated are denatured into single strands (ssDNA).

Figure 1d. Capillary or gel electrophoresis is used to separate the ssDNA fragments of varying sizes/lengths. DNA is negatively charged and will therefore move to the negative electrode. Smaller fragments will travel faster than larger fragments, due to the sieving effect of the separation matrix. The fragments can be resolved to better than the difference of a single nucleotide. By running all tracks on the same gel concurrently, fragments corresponding to their respective nucleotide types can be read out in the size order that they appear, and a sequence may be obtained.

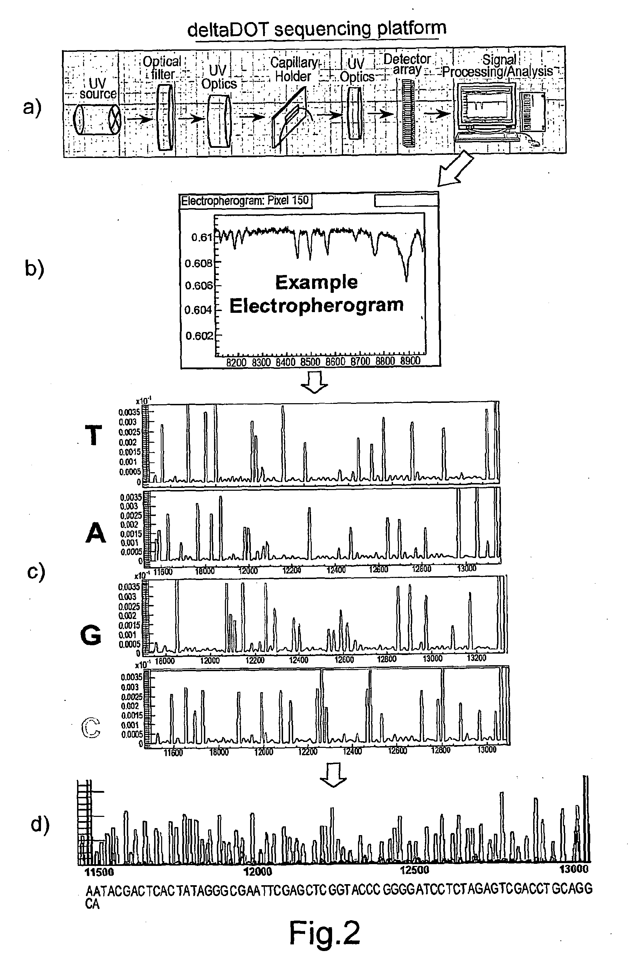

Referring now to Figure 2, this illustrates a sequencing platform which may be used with label free intrinsic imaging, to obtain a DNA sequence without the use of additional labels. The steps represented diagrammatically in this figure demonstrate signal processing and nucleotide discrimination as used to obtain a sequence as briefly indicated in connection with Figure 1d.

Figure 2a. This figure describes the components of our system. Ultraviolet light is focused through a series of filters and optics to a separation capillary. As bands containing the DNA fragments move across the detection window, the drop in UV intensity at 254 nm is measured by a 512 pixel photodiode array detector. We use a matrix consisting of Polyethyleneoxide (PEO) to resolve individual DNA fragments of different sizes, although any suitable matrix may be used, solid or liquid.

Figure 2b. A single electropherogram is depicted in this figure; the troughs seen here represent individual DNA fragments. The photodiode array generates 512 such electropherograms for a single scan.

Figure 2c. These electropherograms are processed using the techniques described in WO02/12877 to reduce background noise and enhance signal intensity tenfold. Other processing techniques may of course be used; all that is necessary is to obtain an output indicating the presence or absence of a DNA band at a particular position. The enhanced EVA (signal processing software) processed output for four individual track runs can be seen plotted here. Marker peaks, DNA fragments of known size and concentration that have been added to all tracks, can be seen at extreme left and right ends of each plot. Markers are added to the four tracks in order to have points of reference common to all tracks for alignment.

Figure 2d. The alignment of the four nucleotide tracks from Figure 2c can be seen superimposed here using sequence alignment software. Again, any suitable software may be used. The markers used in the alignment have been highlighted at the extreme left and right ends of the plot, and the sequence that this alignment produces can be seen below the graph.

### Artefact Peaks

An artefact peak, sometimes known as a shadow band, is generally a loosely defined term for any peak that can be seen in a separation that does not correspond to a correctly sized fragment terminated by the respective ddNTP. Artefact peaks can be subdivided into primer induced artefact peaks and template induced artefact peaks. Primer related artefacts occur when the primer used has an affinity for binding to other regions of the template that it is not intended to bind to leading to the formation of DNA fragments unrelated to the intended sequence.

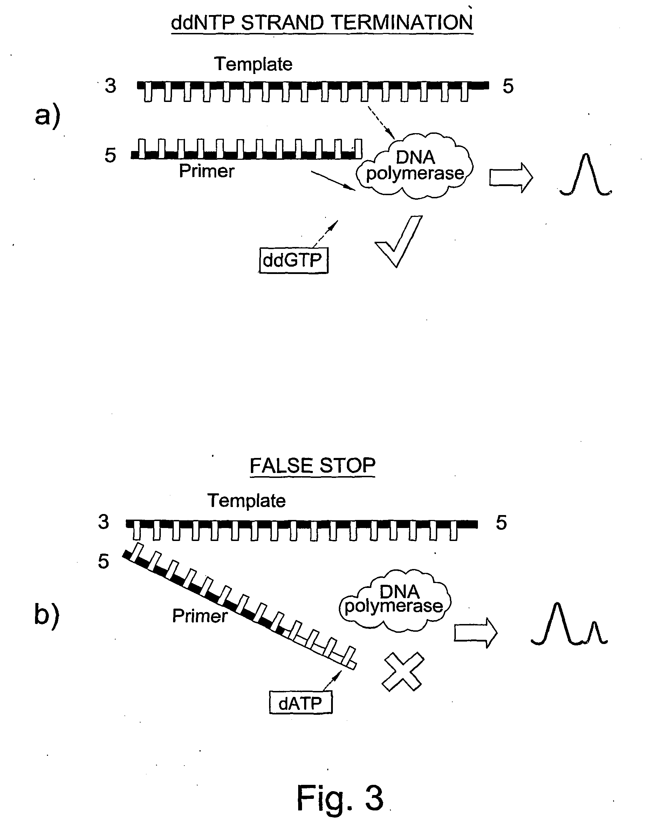

We are more concerned with template related artefacts otherwise known as false stops, or referred to herein as termination artefacts. These peaks are generated as a result of the DNA polymerase falling off the template before a ddNTP has been included (see Figure 3). It is thought that the secondary structure of the template DNA is responsible for this false termination. DNA polymerases also have a finite periodicity in terms of their association to the template, this is called processivity and short processivity frequencies are thought to increase the number of artefacts. All sequence tracks seen in this document are generated using Taq DNA polymerase which has a processivity of approximately 40 bp and are thought not to contain primer associated artifact peaks.

Figure 3a. In a normal round of Sanger chain termination, DNA polymerase is prevented from extending the growing chain when it encounters a ddNTP. Figure 3b. When DNA polymerase dissociates from the template halting DNA chain extension without including a ddNTP the peak generated is called a false stop.

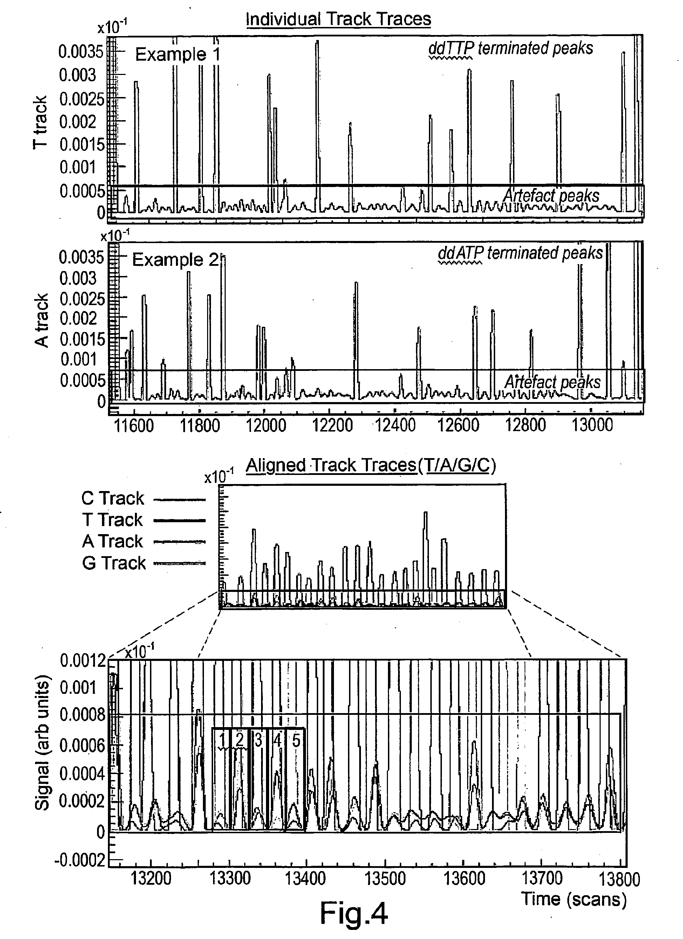

Artefact Peaks or termination artefacts as seen in the sequencing system described in Figure 2 are now discussed, with reference to Figure 4. Due to the excellent signal sensitivity attained by EVA, artefact peaks generated for individual DNA tracks are observable. The comparable track traces shown under the "Individual Track Traces" section in Figure 4 demonstrate the signal processed output that is generated by the sequencing platform. Both tracks (T and A) depict the same stretch of sequence aligned by the large peaks seen at either side of both graphs. Artefact peaks can be seen in the lower portion of the graphs while peaks terminated by the respective ddNTP are seen in the upper portion. It is apparent that the ddNTP peaks are of much greater magnitude than the termination artefact peaks. DNA from each track was electrokinetically injected for 2min at 5Kv and separation was carried out at 14Kv with a 70cm separation to the detection window. The capillary used had an internal diameter of 75µM and 5 Md PEO was used as the separation matrix. 200 cycles of the ddNTP chain termination reaction were carried out, using Taq DNA polymerase.

The second part of Figure 4 (titled "Aligned Track Traces (T/A/G/C)") shows four ddNTP track traces aligned and displayed through our alignment software, TrackAligner. The magnified section shows that complementary artefact peaks between track traces correspond to one another both in peak height and peak morphology. A number of observations have been noted in the figure; 1) artefact peaks can be used to mark individual base pair positions between real ddNTP terminated peaks - this was noted in the original paper for Sanger sequencing (Sanger et al 1977); 2) often the artefact peak preceding a ddNTP peak for a given track will be smaller than expected (This might be a resolution or compression related issue). Therefore, as a rough guide, at any given base pair position for an aligned sequence there will be one large peak representing the ddNTP terminated fragment and three artefact peaks representing an empty base pair position, of the 3 artefact peaks one might be smaller than expected if the next base pair position terminates with its respective track ddNTP so at any given base pair position at least 2 artefact peaks should have similar morphology.

Preliminary results indicate that this artefact correlation is maintained across different ddNTP reactions, through separate experiments.

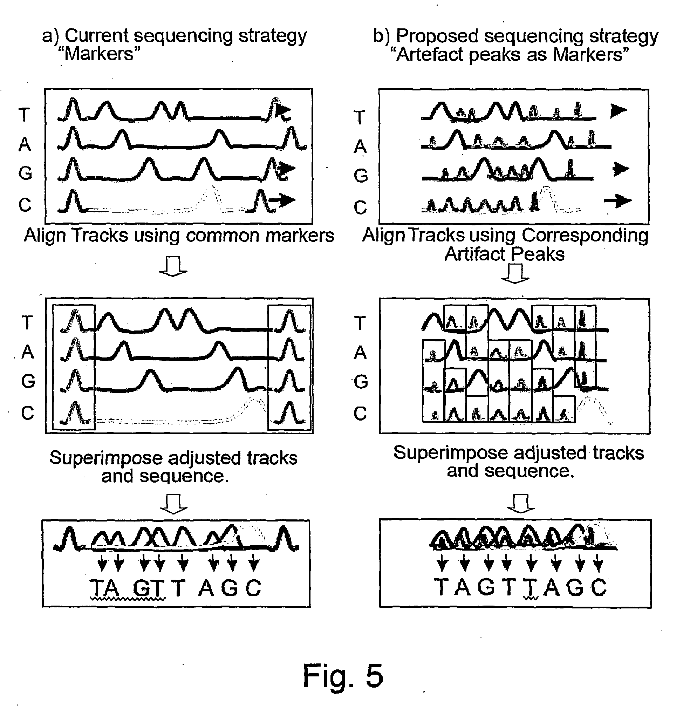

Figure 5 illustrates our proposed DNA sequencing strategy using artefact peaks. Figure 5a. The current sequencing strategy utilises artificially introduced marker peaks (described in Figure 2c). Track traces are stretched and contacted against their marker peaks, the adjusted tracks are merged into one graph and the sequence is determined by reading off the sequence of peaks corresponding to their respective tracks. Figure 5b. We propose a sequencing strategy which would use artefact peaks as intrinsic markers for each track. Corresponding artefact peaks from the different tracks are determined and then used to align the track traces as demonstrated in figure 5. Three corresponding artefact peaks are used to calibrate three tracks at a time for all the corresponding artefact peaks that can be identified. Although all four tracks are not aligned at the same time for each artefact alignment, the four tracks will be aligned progressively as corresponding artefacts are identified between all four tracks. In certain cases, where only two corresponding artefact peaks are identified, two tracks will be calibrated for that base pair position. The combined progressive alignment of the artefact peaks will create an exceptionally good alignment; theoretically if there are identifiable marker peaks for every base pair position for all tracks there will be marker calibration for each base pair position three tracks at a time.

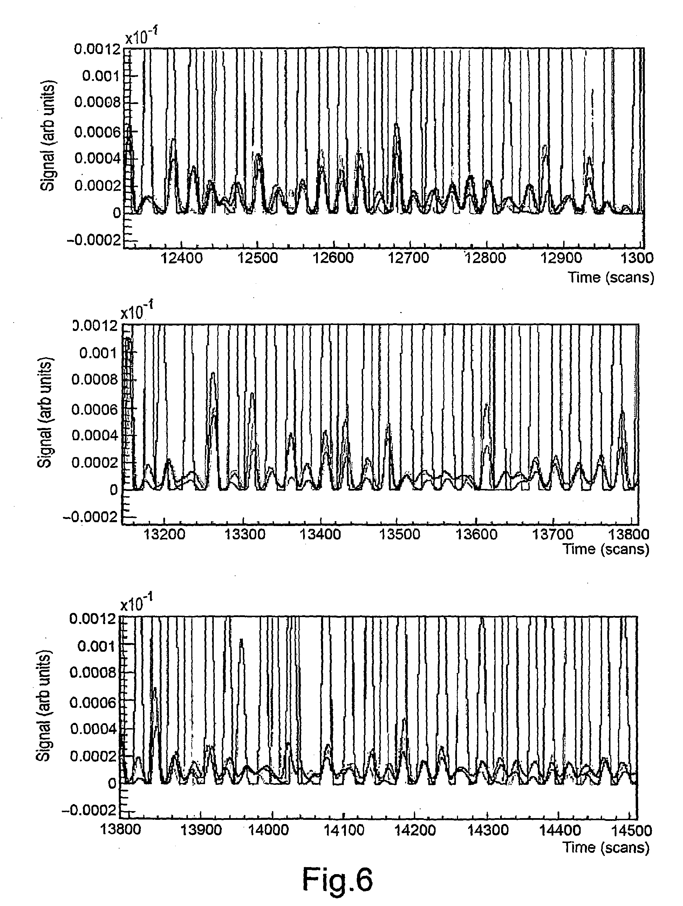

Extended track alignments for the plasmid pGEM3zf(-) DNA sequence are shown in Figure 6. The experimental conditions used to generate these graphs are as described in connection with Figure 4. These graphs demonstrate that although there is homology for artefact peaks between different tracks, certain areas are better than others. Realistically, diminished signal intensity, as well as erroneous DNA fragment contamination may affect the fidelity of corresponding artefact peaks. However, these problems should be overcome programmatically. Moreover as a general rule, there are always at least two tracks whose corresponding artefacts are homologous. Individual artefact peaks will need to be identified using pattern recognition techniques.

## Claims

1. A method of automated sequencing of a nucleic acid using artefact peaks arising from chain termination reactions in Sanger-type nucleic acid sequencing, the method comprising
providing to a sequencing apparatus a plurality of data sets, each set comprising data representing the concentration of synthesised nucleic acids from a plurality of chain termination reactions, wherein the data sets include termination artefact peaks;
identifying termination artefact peaks and aligning two or more of the data sets by correlating one or more termination artefact peak present in said two or more data sets; and determining the nucleic acid sequence from said aligned data sets.

2. The method of claim 1 wherein the nucleic acid is DNA.

3. The method of any preceding claim wherein the concentration of the nucleic acids is represented by the detected intensity of light from the nucleic acids; or by the detected absorption ofUV light by the nucleic acids.

4. The method of any preceding claim wherein each data set represents the concentration of synthesised nucleic acids separated according to a physical characteristic, preferably chain length; optionally each data set including data representing the location of the different nucleic acids within a data set.

5. The method of claim 4 wherein two or more of the data sets are obtained from the same separation.

6. The method of any preceding claim wherein the step of aligning the data sets comprises determining the location of at least one termination artefact present in at least two data sets, and transforming the data sets such that the termination artefacts are present in the same location in each transformed data set.

7. The method of any preceding claim wherein a plurality of termination artefacts are used to align the data sets.

8. The method of any preceding claim wherein the termination artefacts are present in more than two data sets.

9. The method of any preceding claim comprising the step of generating the plurality of data sets;
wherein preferably generating the data sets comprises detecting the concentration of synthesised nucleic acids from a chain termination reaction, optionally by causing the nucleic acids to pass between a light source and a light detector, wherein the nucleic acids are optionally size fractionated, preferably by electrophoresis, before or while they are caused to pass between the source and detector; and including a data item in the data set representing that concentration.

10. The method of any of claims 1 to 8, comprising the steps of fractionating synthesised nucleic acids; passing the fractionated nucleic acids over a detector arranged to detect the concentration of said nucleic acids; and generating a data set representing the concentration of the fractionated nucleic acids.

11. The method of any preceding claim comprising the step of performing one or more chain termination reactions in order to obtain a plurality of synthesised nucleic acids.

12. An apparatus for sequencing nucleic acids, the apparatus comprising
a detector for detecting the concentration of nucleic acids within a plurality of sets of synthesised nucleic acids from a plurality of chain termination reactions;
a data processor programmed to execute computer software to:
derive a plurality of data sets containing data corresponding to said plurality of sets of synthesised nucleic acids;
align two or more of the data sets based on at least one artefact peaks arising from chain termination reactions in Sanger-type nucleic acid sequencing present in said two or more data sets;
and
output a nucleic acid sequence based on the aligned data.

13. A method of correlating a plurality of chain termination reactions carried out in Sanger-type nucleic acid sequencing, the method comprising
providing a plurality of data sets, each set comprising data representing the concentration of synthesised nucleic acids from a plurality of chain termination reactions, wherein the data sets include artefact peaks arising from chain termination reactions in Sanger-type nucleic acid sequencing; and
aligning two or more of the data sets based on at least one termination artefact present in said two or more data sets, to correlate the plurality of reactions.

14. The use of artefact peaks arising from chain termination reactions in Sanger-type nucleic acid sequencing in a method of sequencing nucleic acids; or in a method of aligning data sets representing chain termination reactions; or in a method of correlating data from chain termination reactions.

## Patentansprüche

1. Verfahren zur automatisierten Sequenzierung einer Nukleinsäure unter Verwendung von Artefact-Peaks, die aus den Kettenabbruchreaktionen beim Sequenzieren von Nukleinsäuren nach Sauger entstehen, wobei das Verfahren Folgendes umfasst:
Bereitstellen einer Vielzahl von Datensätzen an einen Sequenzierungsapparat, wobei jeder Satz Daten umfasst, welche die Konzentration von synthetisierten Nukleinsäuren aus einer Vielzahl von Kettenabbruebreaktionen darstellen, worin die Datensätze Abbruchartefakt-Peaks einschließen;
Identifizieren der Abbruchartefakt-Peak, und Alignieren von zwei oder mehr der Datensätze durch Korrelieren von einem oder mehr Abbruchartefakt-Peak(s), der/die in zwei oder mehr Datensätzen anwesend ist/sind; und
Bestimmen der Nukleinsäuzesequenz aus alignierten Datensätzen.

2. Verfahren nach Anspruch 1, worin die Nukleinsäure für DNA steht.

3. Verfahren nach einem der vorangehenden Ansprüche, worin die Konzentration der Nukleinsäuren wie folgt dargestellt wird: durch die detektierte Intensität von Licht aus den Nukleinsäuren; oder durch die detektierte Absorption von UV-Licht durch die Nukleinsäuren.

4. Verfahren nach einem der vorangehenden Ansprüche, worin jeder Datensatz die Konzentration von synthetisierten Nukleinsäuren darstellt, die nach einem physikalischen Charakteristikum, bevorzugt der Kettenlänge, getrennt werden, wobei jeder Datensatz optional Daten einschließt, welche die Stelle der verschiedenen Nukleinsäuren in einem Datensatz darstellen.

5. Verfahren nach Anspruch 4, worin zwei oder mehr der Datensätze aus der gleichen Trennung erhalten werden.

6. Verfahren nach einem der vorangehenden Ansprüche, worin der Schritt zum Alignieren der Datensätze die Bestimmung der Stelle von mindestens einem in mindestens zwei Datensätzen anwesenden Abbruchartefakt umfasst, und Transformieren der Datensätze dergestalt, dass die Abbruchartefakte in jedem transformierten Datensatz an der gleichen Stelle anwesend sind.

7. Verfahren nach einem der vorangehenden Ansprüche, worin eine Vielzahl von Abbruchartefakten zum Alignieren der Datensätze verwendet werden.

8. Verfahren nach einem der vorangehenden Ansprüche, worin die Abbruchartefakte in mehr als zwei Datensätzen anwesend sind.

9. Verfahren nach einem der vorangehenden Ansprüche, das den Schritt zum Generieren der Vielzahl von Datensätzen umfasst;
worin das Generieren der Datensätze bevorzugt Folgendes umfasst: Detektieren der Konzentration von synthetisierten Nukleinsäuren aus einer Kettenabbruchreaktion, optional durch Veranlassen, dass die Nukleinsäuren zwischen einer Lichtquelle und einem Lichtdetektor passieren, worin die Nukleinsäuren, bevorzugt durch Elektrophorese, optional großenfrakrtioniext werden, bevor oder während sie veranlasst werden, zwischen der Quelle und dem Detektor zu passieren; und Einschließen eines Datenpostens im Datensatz, das diese Konzentration darstellt.

10. Verfahren nach einem der Ansprüche 1 bis 8, das die folgenden Schritte umfasst: Fraktionieren synthetisierter Nukleinsäuren; Leiten der fraktionierten Nukleinsäuren über einen Detektor, der zum Detektieren der Konzentration der Nukleinsäuren angeordnet ist; und Generieren eines Datensatzes, der die Konzentration der fraktionierten Nukleinsäuren darstellt.

11. Verfahren nach einem der vorangehenden Ansprüche, das den folgenden Schritt umfasst:
Durchführen von einer oder mehr Kettenabbruchreaktionen, um eine Vielzahl synthetisierter Nukleinsäuren zu erhalten.

12. Apparat zum Sequenzieren von Nukleinsäuren, wobei der Apparat Folgendes umfaßt:
einen Detektor zum Detektieren der Konzentration von Nukleinsäuren in einer Vielzahl von Sätzen aus synthetisieren Nukleinsäuren aus einer Vielzahl von Kettenabbruchreaktionen;
einen Datenprozessor, der zum Ausführen von Computer-Software programmiert ist, um:
eine Vielzahl von Datensätzen mit Daten abzuleiten, die der Vielzahl von Sätzen aus synthetisierten Nukleinsäuren entsprechen;
zwei oder mehr der Datensätze zu alignieren, die auf mindestens einem der aus den Kettenabbruchreaktionen beim Sequenzieren von Nukleinsäuren nach Sanger entstehenden Artefakt-Peak basieren, der in den zwei oder mehr Datensätzen anwesend ist;
und
eine auf den alignierten Daten basierende Nukleeinsäuresequenz auszugeben.

13. Verfahren zum Korrelieren einer Vielzahl von Kettenabbruchreaktionen, die beim Sequenzieren von Nukleinsäuren nach Sanger durchgeführt werden, wobei das Verfahren Folgendes umfasst:
Bereitstellen einer Vielzahl von Datensätzen, wobei jeder Satz Daten umfasst, welche die Konzentration synthetisierter Nukleinsäuren aus einer Vielzahl von Kettenabbruchreaktionen darstellen, worin die Datensätze Artefakt-Peaks einschließen, die aus Kettenabbruchreaktionen beim Sequenzieren von Nukleinsäuren nach Sanger entstehen, und
Alignieren von zwei oder mehr der Datensätze, die auf mindestens einem Abbruchartefakt basieren, das in den zwei oder mehr Datensätzen anwesend ist, um die Vielzahl von Reaktionen zu korrelieren.

14. Verwendung von Astefakt-Peaks, die aus den Kettenabbruchreaktionen beim Sequenzieren von Nukleinsäuren nach Sanger wie folgt entstehen: in einem Verfahren zum Sequenzieren von Nukleinsäuren; oder in einem Verfahren zum Allignieren von Datensätzen, welche Kettenabbruehreaktionen darstellen; oder in einem Verfahren zum Korrelieren von Daten aus den Kettenabbruchreaktionen.

## Revendications

1. Procédé de séquençage automatisé d'un acide nucléique en utilisant des pics d'artefacts provenant de réactions de terminaison de chaîne dans le séquençage d'acides nucléiques de type Sanger, le procédé comprenant:
fournir une pluralité de jeux de données à un appareil de séquençage, chaque jeu comprenant des données représentant la concentration d'acides nucléiques synthétisés provenant d'une pluralité de réactions de terminaison de chaîne, dans lequel les jeux de données comprennent des pics d'artefacts de terminaison;
identifier des pics d'artefacts de terminaison et aligner deux jeux de données ou plus en mettant en corrélation un ou plusieurs pics d'artefacts de terminaison présents dans lesdits deux jeux de données ou plus, et
déterminer la séquence d'acide nucléique desdits jeux de données alignés.

2. Le procédé de la revendication 1, dans lequel l'acide nucléique est un ADN.

3. Le procédé de l'une quelconque des revendications précédentes, dans lequel la concentration des acides nucléiques est représentée par l'intensité détectée de lumière des acides nucléiques ou par l'absorption détectée de lumière UV par les acides nucléiques.

4. Le procédé de l'une quelconque des revendications précédentes, dans lequel chaque jeu de données représente la concentration d'acides nucléiques synthétisés séparés conformément à une caractéristique physique, de préférence la longueur de chaîne; optionnellement chaque jeu de données comprend des données représentant l'emplacement des différents acides nucléiques dans un jeu de données.

5. Le procédé de la revendication 4, dans lequel deux jeux de données ou plus sont obtenus de la même séparation.

6. Le procédé de l'une quelconque des revendications précédentes, dans lequel l'étape d'alignement des jeux de données comprend déterminer l'emplacement d'au moins un artefact de terminaison présent dans au moins deux jeux de données et transformer les jeux de données de telle sorte que les artefacts de terminaison sont présents au même emplacement dans chaque jeu de données transformé.

7. Le procédé de l'une quelconque des revendications précédentes, dans lequel une pluralité d'artefacts de terminaison est utilisée pour aligner les jeux de données.

8. Le procédé de l'une quelconque des revendications précédentes, dans lequel les artefacts de terminaison sont présents dans plus de deux jeux de données.

9. Le procédé de l'une quelconque des revendications précédentes, comprenant l'étape consistant à générer la pluralité de jeux de données;
dans lequel générer les jeux de données comprend de préférence détecter la concentration d'acides nucléiques synthétisés provenant d'une réaction de terminaison de chaîne, optionnellement en faisant passer les acides nucléiques entre une source de lumière et un détecteur de lumière, où les acides nucléiques sont optionnellement fractionnés selon la taille, de préférence par électrophorèse, avant ou pendant qu'on les fait passer entre la source et le détecteur; et inclure dans le jeu de données un poste de donnée représentant la concentration.

10. Le procédé de l'une quelconque des revendications 1 à 8, comprenant les étapes consistant à fractionner les acides nucléiques synthétisés; à faire passer les acides nucléiques fractionnés sur un détecteur arrangé pour détecter la concentration desdits acides nucléiques; et à générer un jeu de données représentant la concentration des acides nucléiques fractionnés.

11. Le procédé de l'une quelconque des revendications précédentes, comprenant l'étape consistant à effectuer une ou plusieurs réactions de terminaison de chaîne afm d'obtenir une pluralité d'acides nucléiques synthétisés.

12. Appareil de séquençage d'acides nucléiques, l'appareil comprenant
un détecteur pour détecter la concentration d'acides nucléiques dans une pluralité de séries d'acides nucléiques synthétisés provenant d'une pluralité de réactions de terminaison de chaîne;
un processeur de données programmé pour exécuter un logiciel informatique pour:
dériver une pluralité de jeux de données contenant des données correspondant à ladite pluralité de séries d'acides nucléiques synthétisés;
aligner deux jeux de données ou plus sur la base d'un pic d'artefact provenant de réactions de terminaison de chaîne dans le séquençage d'acides nucléiques de type Sanger, présent dans lesdits deux jeux de données ou plus;
sortir une séquence d'acide nucléique sur la base des données alignées.

13. Méthode de mise en corrélation d'une pluralité de réactions de terminaison de chaîne effectuées dans le séquençage d'acides nucléiques de type Sanger, la méthode comprenant:
fournir une pluralité de jeux de données, chaque jeu comprenant des données représentant la concentration d'acides nucléiques synthétisés provenant d'une pluralité de réactions de terminaison de chaîne, où les jeux de données comprennent des pics d'artefacts provenant de réactions de terminaison de chaîne dans le séquençage d'acides nucléiques de type Sanger; et
aligner deux des jeux de données ou plus sur la base d'au moins un artefact de terminaison présent dans lesdits deux jeux de données ou plus, afin de mettre la pluralité de réactions en corrélation.

14. Utilisation de pics d'artefacts provenant de réactions de terminaison de chaîne dans le séquençage d'acides nucléiques de type Sanger, dans un procédé de séquençage d'acides nucléiques ou dans une méthode d'alignement de jeux de données représentant des réactions de terminaison de chaîne ou dans une méthode de mise en corrélation de données provenant de réactions de terminaison de chaîne.
